# EUROPEAN PATENT APPLICATION

(11) **EP 3 808 269 A1**
(43) Date of publication of application: **21.04.2021**
(21) Application number: 19203157.3
(22) Date of filing: 15.10.2019
(51) Int. Cl.: A61B 5/117, A61B 5/00, A61B 5/0205

(54) **DEVICE, METHOD AND SYSTEM FOR DETERMINING A VITAL SIGN OF A SUBJECT**

(71) Applicant: Koninklijke Philips N.V., 5656 AG Eindhoven (NL)
(72) Inventor: KIRENKO, Ihor Olehovych, 5656 AE Eindhoven (NL); MENA BENITO, Maria Estrella, 5656 AE Eindhoven (NL); DE HAAN, Gerard, 5656 AE Eindhoven (NL); VERKRUIJSSE, Willem, 5656 AE Eindhoven (NL)
(74) Representative: Philips Intellectual Property & Standards

(57) **Abstract**

The present invention relates to a device for determining a vital sign of a subject, said device (10) comprising a processing unit (34) configured to obtain a detection signal, said detection signal being derived from detected electromagnetic radiation transmitted through or reflected from a surface (28, 30, 32) including a skin portion of the subject (16, 18, 20), said surface (28, 30, 32) being irradiated by an irradiation signal illuminating said surface (28, 30, 32) and carrying an identifier allowing to distinguish said subject (16, 18, 20) from other subjects (16, 18, 20); derive the identifier from the obtained detection signal and identify the subject (16, 18, 20) using the derived identifier; and determine the vital sign from the obtained detection signal and assign the determined vital sign to the identified subject (16, 18, 20).

## Description

### FIELD OF THE INVENTION

The present invention relates to a device, a method and a system for determining a vital sign of a subject.

### BACKGROUND OF THE INVENTION

Traditional care environments require a great deal of manpower. Lately, ward nurses expressed concerns about current monitoring practices and indicated that a patient's condition could destabilize and go unnoticed when, for example, patients walk around in a waiting room or move from one room to another.

Patient assessment and monitoring of vital signs are acknowledged being crucial in supporting ward nurses to follow patients, recognize changes in a patient's condition and react timely to patient deterioration. Thereby, knowledge of the patient enables and facilitates the correct interpretation of vital signs and physiological indicators in the context of each patient and her/his medical history.

Thereby, vital signs of a patient, for example the heart rate (HR), the respiration rate (RR) or the arterial blood oxygen saturation (SpO2), serve as indicators of the current state of the patient and as powerful predictors of serious medical events. For this reason, vital signs are extensively monitored in inpatient and outpatient care settings, at home or in further health, leisure and fitness settings.

One way of measuring vital signs is plethysmography. Plethysmography (PPG) generally refers to the measurement of volume changes of an organ or a body part and in particular to the detection of volume changes due to a cardio-vascular pulse wave traveling through the body of a subject with every heartbeat.

PPG is an optical measurement technique that evaluates a time-variant change of light reflectance or transmission of an area or volume of interest. PPG is based on the principle that blood absorbs light more than surrounding tissue, so variations in blood volume with every heart beat affect transmission or reflectance correspondingly. Besides information about the heart rate, a PPG waveform can comprise information attributable to further physiological phenomena such as the respiration. By evaluating the transmittance and/or reflectivity at different wavelengths (typically red and infrared), the blood oxygen saturation can be determined.

Conventional pulse oximeters (also called contact PPG device herein) for measuring the heart rate and the (arterial) blood oxygen saturation of a subject are attached to the skin of the subject, for instance to a fingertip, earlobe or forehead. Therefore, they are referred to as 'contact' PPG devices. Contact PPG devices may be used for patients that are allowed to walk around in an open ward (e.g. home for elderly) and need monitoring of certain vital signs and current location. Thereby, such patients can, for example, carry a pulse-oximeter on their finger tip, said pulse-oximeter having Wi-Fi or Bluetooth connection in order to transfer determined vital signs to a monitoring system.

However, these devices can be cumbersome, since they have detectors, processing electronics and communication electronics, which all require power. Furthermore, even though contact PPG is regarded as a non-invasive technique, contact PPG measurement is often experienced as being unpleasant and obtrusive, since the pulse oximeter is directly attached to the subject and any cables limit the freedom to move and might hinder a workflow.

Thus, non-contact, remote PPG (rPPG) devices (also called camera-based device) for unobtrusive measurements have been proposed in the last decade. Remote PPG utilizes light sources or, in general radiation sources, disposed remotely from the subject of interest. Similarly, also a detector, e.g., a camera or a photo detector, can be disposed remotely from the subject of interest. Therefore, remote photoplethysmographic systems and devices are considered unobtrusive and well suited for medical as well as non-medical everyday applications.

As an example, Verkruijsse et al., "Remote plethysmographic imaging using ambient light", Optics Express, 16(26), 22. Dec. 2008, pp. 21434-21445 demonstrates that photoplethysmographic signals can be measured remotely using ambient light and a conventional consumer level video camera, using red, green and blue color channels.

Alternative to a completely non-contact rPPG, it is also possible to attach a wearable light source to a portion of a patient's body, for example behind an earlobe or on one side of a hand of the patient, while still observing the patient with a remotely arranged camera. Such a system is shown, for example, in US 2015/0223700 A1. Even though such identification may be suitable for monitoring a specific patient even when she/he walks in an open space or changes from one room to another, this monitoring technique may be unsuitable in open wards and/or when multiple patients need to be monitored at once, since in such conditions it is not obvious to distinguish between multiple subjects.

Thus, the above-mentioned vital sign supervision techniques have some drawbacks, since recognition and continuous monitoring of a specific patient, especially in a group of multiple patients and/or in spaces where patients are allowed to walk around freely, can be difficult.

In order to address this problem, said patient assignment may be based on face detection which is used to identify a specific patient to be monitored out of a group of patients. By knowing the identity of said patient and by including further information (such as health history), it may be possible to assign a specific vital sign which has been detected among a plurality of vital signs of multiple patients to the identified patient. One such identification method may be found in US 2018/300540 A1, where both face related and non-face related features are used by a machine-learning model for identifying individuals in a waiting room.

However, such identification may not always work, for example, when bandages or other medical accessories are occluding the face. Furthermore, this kind of identification does not consider privacy of a patient, which has become an important subject in latest times.

### SUMMARY OF THE INVENTION

It is an object of the present invention to provide a device, a method and a system with which a vital sign of a subject can be monitored even in a group of subjects or while the subject moves freely. Furthermore, it is an object of the present invention to avoid face recognition for identifying the subject to be monitored.

In a first aspect of the present invention a device for determining a vital sign of a subject is presented. Said device comprises a processing unit configured to obtain a detection signal, said detection signal being derived from detected electromagnetic radiation transmitted through or reflected from a surface including a portion of skin of the subject, said surface being irradiated by an irradiation signal illuminating said surface and carrying an identifier allowing to distinguish said subject from other subjects, to derive the identifier from the obtained detection signal and identify the subject using the derived identifier; and to determine the vital sign from the obtained detection signal and assign the determined vital sign to the identified subject.

In a further aspect of the present invention a system for determining a vital sign of a subject is presented. Said system comprises a light source configured to emit an irradiation signal to a surface including a portion of skin of the subject, said irradiation signal carrying an identifier allowing to distinguish said subject from other subjects, a detection unit configured to contactless detect electromagnetic radiation transmitted through or reflected from the surface including the portion of skin of the subject of the subject and to derive a detection signal from said electromagnetic radiation; and a device according to the present invention configured to determine the vital sign of the subject from the obtained detection signal.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea that with the proposed intelligent monitoring device care work force can be reduced especially for care places in open wards. With the device according to the present invention, a current location of the subject in a care place (e.g. in a ward place, elderly care place or in a waiting room) can be obtained continuously, since the current location of the subject may be preferably extracted from the detection signal. Furthermore, the derived identifier unambiguously identifies the subject and makes it possible that a certain determined vital sign, out of a plurality of detected vital signs can be assigned to the identified subject so that it can be monitored whether an abnormal event occurs.

According to the invention, assignment of a vital sign to a respective subject is done via the identifier, which is included in the obtained detection signal. Thus, no facial recognition is necessary and privacy of the subject can be maintained. Furthermore, it is possible to monitor subjects whose faces are covered for example with bandages or other medical equipment. Thus, patients may be allowed to freely move from one ward to another.

Another advantage is that due to the dual use of the detection signal, not only vital sign extraction of a subject is possible. In addition, identification of a subject to be monitored out of a group of multiple subjects is possible by means of the identifier. The identifier may include predefined signal information that can be uniquely used to identify the subject to be monitored.

A further advantage is that with the device, the system and the method of the present invention multiple subjects can be jointly monitored at once by using multiple identifiers, each of which identifies exactly one subject.

The detection signal according to the present invention comprises electromagnetic radiation of one or more wavelengths or wavelength ranges which was transmitted through or reflected from the surface of the subject, which surface being preferably illuminated by light of one or more predefined wavelengths or wavelength ranges. The light for illuminating the surface including the portion of skin of the subject is defined as the irradiation signal. The detection signal may carry information about one or more vital signs of the subject. Furthermore, the detection signal preferably includes predefined information, namely the identifier, resulting from a modulation and/or coding of the light represented by the irradiation signal.

Thus, according to the present invention, the detection signal has a dual function, since it is used both for identifying the subject by means of the identifier and for deriving a PPG-based vital sign with the identified subject.

The illuminated surface of the subject can, for example, be a selectively illuminated part of a skin surface (epidermis) together with its underlying skin layers (e.g. dermis). The reflected electromagnetic radiation may not only contain a contribution of electromagnetic radiation reflected from the skin portion of the subject but also electromagnetic radiation reflected from non-skin portions, such as cloth, bandages and/or medical equipment worn by the subject. Only the electromagnetic radiation reflected from the skin portion of the subject and being included in the detection signal contains the pulsatile and non-pulsatile compounds used for the determination of one or more vital signs of the subject. However, the electromagnetic radiation reflected from a non-skin portion of the subject and being included in the detection signal may (alone or in addition) be used to derive the identifier and thus, to identify the subject using the derived identifier.

In a further embodiment of the device according to the present invention, the processing unit is configured to compare the derived identifier with a database correlating different identifiers with different subjects. An advantage of this embodiment is that the database can be outsourced from the device and may be part of a centralized hospital monitoring and health care system. In such case, if a new subject is to be monitored, a new identifier for identifying the new subject can be added manually or automatically to the database without the need of retrofitting an existing system. Additionally, the database may include further subject-related health and/or private information, such as family members or relatives to be informed if an extraordinary medical event occurs. Thus, doctors or other medical staff may easily derive further subject-related information if necessary. It is preferable that an alert device is equipped for generating an alert if the device detects an abnormal change in the detected vital sign of the subject. Furthermore, a health record of the subject, preferably included in the database, may be used to output an urgency of said alert (e.g. if the subject showing the abnormal change in the detected vital sign already had a heart attack).

In a further embodiment of the device according to the present invention, the processing unit is configured to derive the identifier in the irradiation signal by identifying one or more of a selected frequency of the detected electromagnetic radiation, a predefined frequency pattern of the detected electromagnetic radiation, a selected phase or phase-pattern of the detected electromagnetic radiation, a selected amplitude of the detected electromagnetic radiation, and a modulation of a selected frequency, a predefined frequency pattern, a selected phase or phase-pattern and/or a selected amplitude of the detected electromagnetic radiation. The selected frequency of the detected electromagnetic radiation is preferably a frequency of a modulated electromagnetic radiation signal. Preferably, the frequency modulation (FM) may be based on direct current (DC) FM, exponential FM, linear FM or linear through ZERO FM. The selected amplitude is preferably defined as the relative amplitude of the detected electromagnetic radiation, since an absolute amplitude determination might be unreliable, since it may depend on a distance to and/or a color of the skin of the subject. Thus, by means of the relative amplitude, these disturbing parameters can be balanced out. In other words, identification of the subject can be done by means of coded and/or modulated light (for example light having predefined frequency, pattern and/or color).

For example, a predefined frequency pattern (such as used in a lighthouse) may be used to increase the number of possible identifiers and with it the number of identifiable subjects. Furthermore, it may be possible to use two differing frequencies of two differing wavelength included in the electromagnetic radiation which may also be used to extract, for example, SpO2 concentration as the identifier. Thus, the number of possible identifiers can be further increased. With an increased number of identifiers, a group of subjects to be identified and monitored can be larger. Thus, large wards or hospitals with tens or hundreds of subjects may be monitored.

Alternatively or supplementary to the use of a selected frequency, phase and/or amplitude being applied to a wavelength or a wavelength range which is used for vital sign detection, it is preferable to use a predefined wavelength as the identifier which is not used for vital sign detection, but which is derivable from the detection signal (e.g. visible for a camera used as a detection unit) to identify the subject. Furthermore, it may be possible to use a wavelength or wavelength range, which is used for vital sign detection but may be different for each subject in order to allow identification of the subject. It is preferable to combine these options for identifying the subject with the aforementioned frequency, frequency pattern, phase, phase-pattern and/or relative amplitude modulation to enable a larger number of subjects to be monitored at once.

Furthermore, it may be preferable to include the identifier directly in the electromagnetic radiation. If, for example, band pass filters with different central wavelengths were used in front of LEDs, or even LEDs having differing wavelengths or wavelength ranges were used, it may be possible to identify a certain wavelength of the electromagnetic radiation (spectrometer) as the identifier.

In a further embodiment of the device according to the present invention, the processing unit is configured to derive the identifier from the obtained detection signal by identifying a selected frequency outside the frequency range from 0.5 to 3 Hz of the detected electromagnetic radiation. This embodiment enables that code frequencies used as identifier can be clearly distinguished by the processing unit.

In a further embodiment of the device according to the present invention, the processing unit is configured to derive the identifier from the obtained detection signal by identifying a selected frequency in a frequency range below or at 50 Hz, in particular below or at 15 Hz. This is advantageous, since most state of the art cameras operate at frame rates up to 50 Hz, but typically at 15 Hz. However, also a higher frame rate boundary is possible and with it a higher frequency boundary (i.e. not up to 50 Hz but rather up to 1000 Hz). A higher frequency boundary has the advantage that a higher number of subjects can be identified. Alternatively, a parallel camera setup having one camera with a low spatial resolution but therefore a high frame rate (e.g. > 1000 Hz) and another camera having a high resolution but therefore a low frame rate (< 50 Hz, typically 15 Hz) may be used for subject localization and vital sign extraction. Here, the camera with low resolution but high frame rate can be used to localize the subject, whereas the camera with high resolution but low frame rate can be used for vital sign extraction.

In a further embodiment of the device according to the present invention, the processing unit is configured to derive the identifier from the obtained detection signal by identifying a predefined frequency pattern or phase pattern carried in the irradiation signal, in particular by alternately switching on and off of a selected phase-delay, frequency or frequency range. This embodiment has the advantage that the number of possible patterns is high and with it the number of subject being identified according to a predefined pattern. The pattern can be symmetrical or asymmetrical. In a case where the pattern is symmetrical, it preferably shows equal signal time intervals and equal non-signal time intervals. If the pattern is asymmetrical, the signal time intervals and/or the non-signal time intervals can be of different lengths / duration.

In a further embodiment of the device according to the present invention, the processing unit is configured to derive the identifier from the obtained detection signal by identifying a selected phase carried in the irradiation signal. In this case, the processing unit is preferably configured to determine the selected phase of the modulated/coded irradiation signal by, for example, comparing a predefined reference phase with the selected phase of the irradiation signal. Thus, the phase does not need to be switched in different parts of the irradiation signal, which may however be a further option.

In a further embodiment of the device according to the present invention, the processing unit is configured to derive the identifier from the obtained detection signal by identifying a selected amplitude which is preferably the relative amplitude carried in the irradiation signal. The relative amplitude may be the normalized amplitude or may define an amplitude range having amplitude bounds within which a true amplitude of the signal is supposed to lie. The amplitude may be modified by changing the detection signal, e.g. by enhancing the signal.

In a further embodiment of the device according to the present invention, the processing unit is communicatively connected to a light source for irradiating a surface including a portion of skin of the subject and configured to control the light source to emit an irradiation signal carrying the identifier. This embodiment has the advantage that the processing unit can communicate with the light source directly or indirectly via, for example, a central controller. This embodiment enables the processing unit to control the light source such that the irradiation signal is modified or coded by means of the predefined identifier. Thus, it may be possible, when a new subject needs to be monitored, to predefine a new identifier for said new subject within the database and control a standardized light source such that its emitted irradiation signal carries said predefined identifier. The communication between the light source and the processing unit is preferably wireless and auto controlled (e.g. via an online database synchronization).

In a further embodiment of the device according to the present invention, the processing unit is further configured to determine a current location of the subject on the basis of a predetermined location of a detection unit and the detection signal. The location of the detection unit may be predetermined during a calibration procedure and may be a spatial reference location in a room or yard. The location of the detection unit may be a camera location. Based on a reference camera location and preferably based on further temporal information and/or information on the direction and angle of incidence of electromagnetic radiation transmitted through or reflected from the surface including a portion of skin of the subject, the exact location of the subject can be detected. This embodiment has the advantage that by means of the predetermined camera location, a current location of the subject is known continuously which enables monitoring of the subject while the subject is moving.

In a further embodiment of the system according to the present invention, the detection unit is a camera configured to acquire a set of image frames of a region of interest representing a whereabouts of the subject. The region of interest may be a room or a yard but may also be a public or private open space, for example, a park or a garden. The camera may be a camera with preferably a high resolution, for example, a frame rate of up to 50 Hz. However, cameras with lower resolution but a higher frame rate may be suitable. Various cameras may be used to observe the whereabouts from different directions.

In a further embodiment of the system according to the present invention, the light source is a wearable light source configured to be worn at a portion of a subject's body, in particular behind an ear or on one side of a hand of a subject. It should be understood that the forgoing listing of portions of a subject's body should be understood exemplarily and other body portions of the subject, such as a forehead, a foot or any other anatomical location, may be included herewith as well. It is preferable that the subject wears the wearable light source at a body portion having a soft tissue and thus, more efficiently transmits the electromagnetic radiation. Earlobes or fingertips may represent suitable portions of a subject's body. For example, a subject may be equipped with a wearable LED light source which is to be worn by the subject and may comprise additional electronics to emit electromagnetic radiation at identifiable frequencies, wavelengths and/or patterns. Thus, said LED can be uniquely identified without the need of face detection of the subject that wears it, while camera-based vital signs measurement, using the light emitted from the wearable light source and being transmitted or reflected from the surface including a portion of skin of the subject is not hindered by the frequency, wavelength or pattern used for the identification of the subject.

In a preferred embodiment of the system, live views from the detection unit (e.g. a camera) can be (wirelessly) transferred to caregiver staff with alerting vital sign values superimposed, and/or the heart rate for each monitored individual subject can be logged and/or viewed in a subject / patient database. This has the advantage that caregiver staff is able to visually evaluate certain situations in which vital signs of one or more subjects show alerting values.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of a system according to the present invention;
Fig. 2 shows a flowchart of a method according to the present invention;
Fig. 3 shows a flowchart of an exemplified probe identification and vital sign assignment according to the present invention;
Fig. 4 shows an example of a database correlating different identifies with different subjects;
Figs. 5A and 5B show schematic diagrams of a state of the art irradiation and detection signal, respectively;
Figs. 6A and 6B show schematic diagrams of an irradiation and a detection signal according to the present invention, respectively; and
Figs. 7A and 7B show two exemplary detection signals with two different frequency patterns.

### DETAILED DESCRIPTION OF EMBODIMENTS

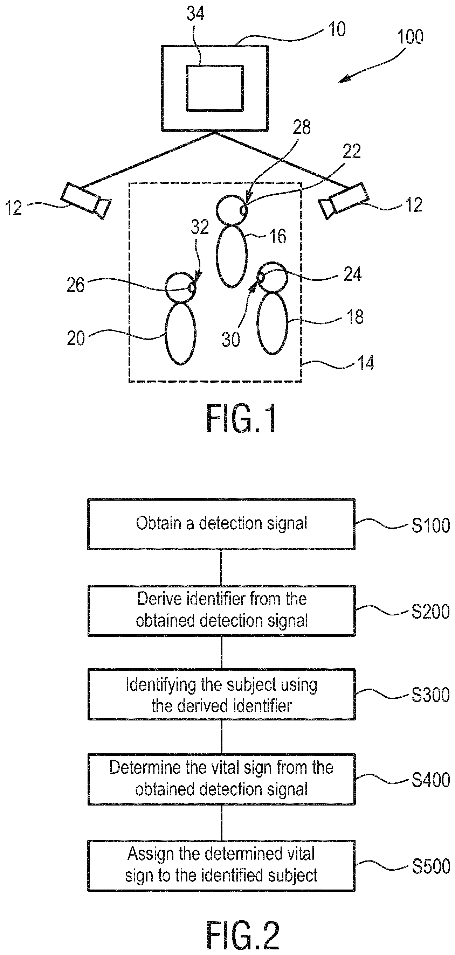

Fig. 1 shows a schematic diagram of an embodiment of a system 100 according to the present invention. The system 100 comprises a device 10 and a detection unit 12. In Fig. 1, the system 100 comprises two detection units 12 which are represented by two cameras. The cameras 12 are each configured to acquire a set of image frames of a region of interest representing a whereabouts 14 of a subject 16. The cameras in Fig. 1 acquire the region of interest from different angles of view and thus may different perspectives.

The whereabouts 14 of Fig. 1 is a waiting room in a hospital but may be a yard or a hallway in a hospital or medical station or a public or private space or generally any whereabouts of a subject. In the waiting room 16, three individual subjects 16, 18, 20 are present in this example. Each subject 16, 18, 20 is equipped with a wearable light source 22, 24, 26 each of which is configured to be worn at a respective surface 28, 30, 32 including a portion of skin of the subject's 16, 18, 20 body. In Fig. 1 each subject 16, 18, 20 wears the wearable light source 22, 24, 26 behind an ear, in particular at an earlobe, respectively.

The wearable light source 22, 24, 26 may be a LED light source configured to be attachable to the surface 28, 30, 32 including a portion of skin of the subject 16, 18, 20, for example by means of adhesive bonding or clamping. The wearable light source may comprise a lithium-based battery or any suitable battery for enabling the light source to emit electromagnetic radiation at a predefined wavelength or wavelength range. The light source may also comprise two or more LEDs emitting electromagnetic radiation at different wavelengths or wavelength ranges (e.g. red and infrared light). Thus, not only extraction of a heart rate of the to be monitored subject(s) as a vital sign is possible but also further vital signs, such as SpO2 is possible.

The device 10 further comprises a processing unit 34. The processing unit 34 may be a processor or a computer. The processing unit may be integrated in the device 10 or may be connected to the device via one or more cables or wirelessly. The processing unit 34 is configured to obtain (see step S100 of Fig. 2) a detection signal detected from the detection unit 12. The processing unit is preferably configured to obtain multiple detection signals all detected from the detection unit 12. In Fig. 1, the processing unit is configured to obtain three different detection signals from the three monitored subjects 16, 18, 20. Each detection signal being derived from detected electromagnetic radiation transmitted through or reflected from the respective surface 28, 30, 32 including a portion of skin of the respective subject 16, 18, 20. Each surface 28, 30, 32 including a portion of skin of each subject 16, 18, 20 is irradiated by an irradiation signal emitted from the respective light source 22, 24, 26. Each light source 22, 24, 26 illuminates the respective surface 28, 30, 32 including a portion of skin at which it is attached.

Each irradiation signal of each light source carries a unique identifier allowing distinguishing the respective subject 16, 18, 20 from the other subjects 16, 18, 20 by means of the identifier. For example, the irradiation signal emitted from the light source 22 carries an identifier representative for the subject 16 wearing the wearable light source 22.

The processing unit 34 is further configured to derive the identifier from the obtained detection signal (see step S200 of Fig. 2) and to identify the subject using the derived identifier (see S300 of Fig. 2). Furthermore, the processing unit 34 is configured to determine a vital sign (for example the heart rate) of the subject 16, 18, 20 from the obtained detection signal (see step S400 of Fig. 2) and assign the determined vital sign to the identified subject 16, 18, 20 (see step S500 of Fig. 2). In Fig. 1, the processing unit 34 is configured to derive three different identifiers from the three obtained detection signals, wherein each identifier unambiguously represents one of the three monitored subjects 16, 18, 20. Furthermore, the processing unit 34 is configured to determine three different vital signs from the three obtained detection signals. Thus, at least a part of each obtained detection signal being received in transmission mode is used as a PPG signal for vital sign extraction.

The identifier included in the irradiation signal can be used to identify a certain subject (e.g. the subject 16) out of a group of subjects 16, 18, 20. Therefore, detection of the detection signal(s) e.g. via the camera(s) 12 is possible at a distance and provides not only one or more vital signs of the subject(s) (such as SpO2 and pulse rate) but also information to associate these vital signs with a certain subject only by means of the identifier. After assigning the detected vital signs with the respective subject, vital sign related information can be further processed. On basis of this, the system 100 may provide feedback to caregivers by, for example, sending an alert to a smartphone of caregivers or by monitoring said vital sign related information on a general display unit.

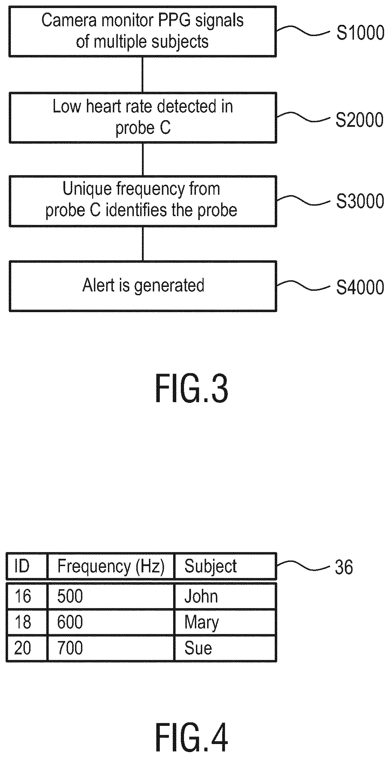

Fig. 3 shows a flowchart of an exemplified probe identification method and vital sign assignment according to a refinement of the present invention. For explanation of Fig. 3, references are made to the exemplary system shown in Fig. 1.

In step S1000, the cameras 12 obtain a PPG-signal based vital sign included in one of the detection signals resulting from electromagnetic radiation being transmitted or reflected from the surfaces 28, 30, 32 including a portion of skin of one of the subjects 16, 18, 20. In step S1000 the processing unit obtains the detection signal and determines said vital sign without knowing of which subject the determined vital sign is.

In step S2000, the processing unit 34 may further determine that said vital sign represents a low heart rate of one of the subjects of the group of subject 16, 18, 20.

Since the detection signal not only includes the vital sign related information but also the identifier, the processing unit 34 is able to identify, for example, the subject 16 of the group of subjects 16, 18, 20 in step S3000 and assign the determined vital sign (namely the low heart rate) to the subject 16 without any need for face recognition of the subject 16. The identifier may be represented as a preselected frequency pattern resulting from a predefined modulation of the irradiation signal of the light source 22.

In step S4000, an alert is generated by, for example, displaying a live view of the cameras 12 for displaying a visual state of the identified subject 16. The alert may be displayed together with the determined heart rate of the subject 16. The alert may be displayed on a regular display, a tablet or a smartphone or through augmented reality means.

Fig. 4 shows an example of a database 36 which is preferably used by the processing unit 34 to compare the derived identifier with its respective subject. Thus, the database may correlate different identifiers with different subjects. In Fig. 4, the database excerpt is represented as a table having three columns and four lines. In the first column, different identifications (IDs) for different subjects are represented. In column 2, a specific identifier for each ID is represented. In Fig. 4, the identifiers are selected frequencies measured in Hz. The database shown in Fig. 4 correlates each ID and each identifier with a corresponding personality being presented as a surname for each subject. As an example, the subject 16 has the identifier 500 Hz and the surname John. Due to this information, it is possible to unambiguously identify the subject by means of the identifier and with it to assign the detected vital sign with the identified subject. The database may be a SQL-based database or any suitable database structure. The database may be stored on a readable non-transitory memory of a computer or server or may be stored in an accessible cloud. The database may be a table-based database but may also show a more complex data structure. Database maintenance and/or actualization can preferably be done manually or automatically, for example, via frequently actualizing the database with online reference data.

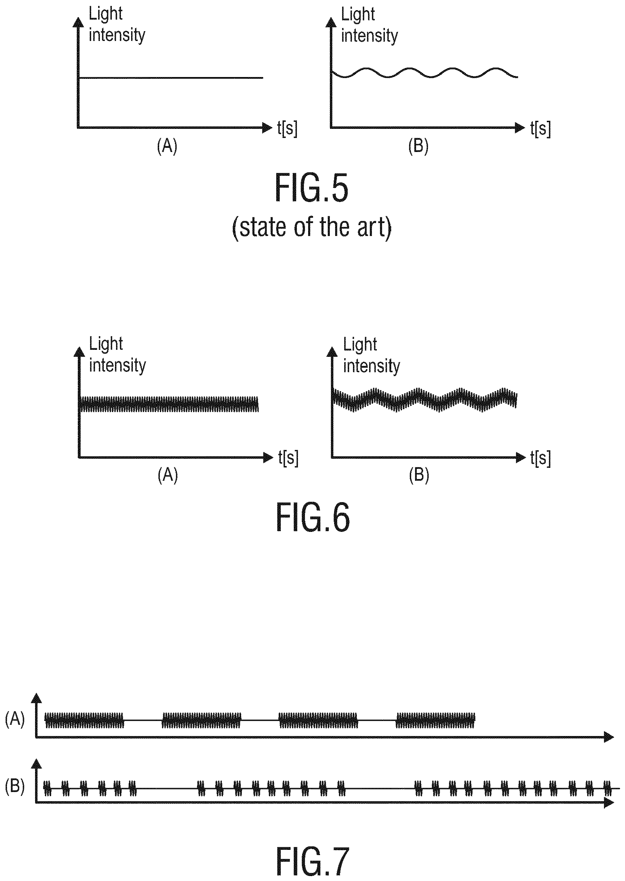

Fig. 5A shows a schematic diagram representing an irradiation signal resulting from electromagnetic radiation and being used for vital sign detection. The presented irradiation signal is shown before the electromagnetic radiation interacts with a skin portion of a subject. Such irradiation signals may be used in the state of the art, for example, in US 2016/174887 A. The ordinate axis of the diagram of Fig. 5A and 5B represents the time in seconds, whereas the abscissa shows light intensity without any specific unit. As shown in Fig. 5A, light intensity of the electromagnetic radiation is constant over time, since the irradiation signal is not modulated or coded with an identifier.

Fig. 5B shows a state of the art detection signal without any additional identifier. The detection signal as shown in Fig. 5B may be presented as such after the interaction (transmittance of reflectance) of the electromagnetic radiation with the surface including a portion of skin of a subject. Thus, the detection signal shows pulsatile characteristics being used for vital sign extraction (for example, the heart rate of the subject). However, with such a detection signal, identification of a subject is not possible.

Fig. 6A shows a schematic diagram representing a modulated and/or coded irradiation signal resulting from modulated/coded electromagnetic radiation and being used for vital sign detection and for identification of a subject by means of the identifier. The identifier is represented as the modulated/coded part of the irradiation signal. The presented modulated/coded irradiation signal is shown before the electromagnetic radiation interacts with a surface including a portion of skin of a subject. The ordinate axis of the diagram of Fig. 6A and 6B represents the time in seconds, whereas the abscissa shows light intensity without any specific unit. As can be seen in Fig. 6A, the identifier is represented by a selected frequency of the light intensity of the electromagnetic radiation. This identifier, namely the selected frequency, is also present in the detection signal of Fig. 6B and can be used to identify a certain subject. The identifier is thus modulated onto the basic irradiation signal (such as the one of Fig. 5A) and is still present in the detection signal shown in Fig. 6B after transmission and/or reflectance of the irradiation signal of Fig. 6A. Thus, identification of a subject based on the identifier is possible.

It may for example be possible to remove the coded / modulated signal part (e.g. frequency (ies)) by means of a low pass filter in order to provide a signal for further PPG (vital sign) extraction. Additionally, by means of a high pass filter, the coded / modulated signal part representing the identifier can be kept in order to use it for identifying the subject.

Figs. 7A and 7B show two exemplary possibilities for irradiation signal modulation. Thereby, according to Fig. 7A, the identifier is represented as a selected frequency pattern which is modulated onto the irradiation signal, for example, by instructing the light source to switch on and off according to a predefined pattern. In the case of Fig. 7A, the frequency pattern is symmetrical and shows equal signal and non-signal durations. In contrast, according to Fig. 7A, the frequency pattern is asymmetrical and therefore shows unequal signal and non-signal durations.

The invention extends the value of camera-based patient-monitors. The contactless monitoring, with a camera, is assumed to be highly relevant for premature babies with very sensitive skin in NICUs, and for patients with damaged (e.g. burns) skin, but may also be more convenient than contact sensors as used in the general ward since contact probes are wired and can squeeze the finger uncomfortably when worn 24/7. Moreover, wired contact probes are a source of worry during a so-called "flying moment" when babies are transferred from the incubator to a mother/father for skin-to-skin contact care (also known as kangaroo mother care). The current invention, since it provides an unwired light source attached to the baby's body portion (for example, foot or hand), is convenient for this "flying moment", as the baby is taken away from the incubator with dedicated illumination for camera-based vital sign extraction (vital sign measurement).

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/ distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device for determining a vital sign of a subject (16, 18, 20), said device (10) comprising a processing unit (34) configured to:
- obtain a detection signal, said detection signal being derived from detected electromagnetic radiation transmitted through or reflected from a surface (28, 30, 32) including a portion of a skin of the subject (16, 18, 20), said surface (28, 30, 32) being irradiated by an irradiation signal illuminating said surface (28, 30, 32) and carrying an identifier allowing to distinguish said subject (16, 18, 20) from other subjects (16, 18, 20);
- derive the identifier from the obtained detection signal and identify the subject (16, 18, 20) using the derived identifier; and
- determine the vital sign from the obtained detection signal and assign the determined vital sign to the identified subject (16, 18, 20).

2. Device according to claim 1, wherein the processing unit is configured to compare the derived identifier with a database (36) correlating different identifiers with different subjects (16, 18, 20).

3. Device according to claim 1 or 2, wherein the processing unit (34) is configured to derive the identifier in the irradiation signal by identifying one or more of
a selected frequency of the detected electromagnetic radiation,
a predefined frequency pattern of the detected electromagnetic radiation,
a selected phase or phase-pattern of the detected electromagnetic radiation,
a selected amplitude of the detected electromagnetic radiation, and
a modulation of a selected frequency, a predefined frequency pattern, a selected phase or phase-pattern and/or a selected amplitude of the detected electromagnetic radiation.

4. Device according to any preceding claim, wherein the processing unit (34) is configured to derive the identifier from the obtained detection signal by identifying a selected frequency outside the frequency range from 0.5 to 3 Hz. of the detected electromagnetic radiation.

5. Device according to any one of claims 1 to 3, wherein the processing unit (34) is configured to derive the identifier from the obtained detection signal by identifying a selected frequency in a frequency range below or at 50 Hz, in particular below or at 15 Hz.

6. Device according to any one of claims 1 to 3, wherein the processing unit (34) is configured to derive the identifier from the obtained detection signal by identifying a predefined frequency pattern or phase pattern carried in the irradiation signal, in particular by alternately switching on and off of a selected phase-delay, frequency or frequency range.

7. Device according to any one of claims 1 to 3, wherein the processing unit (34) is configured to derive the identifier from the obtained detection signal by identifying a selected phase carried in the irradiation signal, in particular by a predefined displacement of phase of at least a part of the irradiation signal.

8. Device according to any one of claims 1 to 3, wherein the processing unit (34) is configured to derive the identifier from the obtained detection signal by identifying a selected amplitude carried in the irradiation signal.

9. Device according to any preceding claim, wherein the processing unit (34) is communicatively connected to a light source (22, 24, 26) for irradiating a surface (28, 30, 32) including a portion of skin of a subject (16, 18, 20) and configured to control the light source (22, 24, 26) to emit an irradiation signal carrying the identifier.

10. Device according to any preceding claim, wherein the processing unit (34) is further configured to determine a current location of the subject (16, 18, 20) on the basis of a predetermined location of a detection unit (12) and the detection signal.

11. System for determining a vital sign of a subject, said system comprising:
- a light source (22, 24, 26) configured to emit an irradiation signal to a surface (28, 30, 32) including a skin portion of a subject (16, 18, 20), said irradiation signal carrying an identifier allowing to distinguish said subject (16, 18, 20) from other subjects (16, 18, 20);
- a detection unit (12) configured to contactless detect electromagnetic radiation transmitted through or reflected from a surface (28, 30, 32) including a skin portion of the subject (16, 18, 20) and to derive a detection signal from said electromagnetic radiation; and
- a device (10) according to one of claims 1 to 10 configured to determine the vital sign of the subject (16, 18, 20) from the obtained detection signal.

12. System according to claim 11, wherein the detection unit (12) is a camera configured to acquire a set of image frames of a region of interest representing a whereabouts of the subject (16, 18, 20).

13. System according to any of claims 11 to 12, wherein the light source (22, 24, 26) is a wearable light source configured to be worn at a portion of a subject's body, in particular behind an ear or on one side of a hand of the subject (16, 18, 20).

14. Method for determining a vital sign of a subject, said method comprising the steps of:
- obtaining (S100) a detection signal, said detection signal being derived from detected electromagnetic radiation transmitted through or reflected from a surface (28, 30, 32) including a skin portion of the subject (16, 18, 20), said surface (28, 30, 32) being irradiated by an irradiation signal illuminating said surface (28, 30, 32) and carrying an identifier allowing to distinguish said subject (16, 18, 20) from other subjects (16, 18, 20);
- deriving (S200) the identifier from the obtained detection signal;
- identifying (S300) the subject (16, 18, 20) using the derived identifier;
- determining (S400) the vital sign from the obtained detection signal; and
- assigning (S500) the determined vital sign to the identified subject (16, 18, 20).

15. Computer program comprising program code means for causing a computer to carry out the steps of the method as claimed in claim 14 when said computer program is carried out on the computer.
